Europäisches Patentamt
European Patent Office
Office européen des brevets

(19)

(11) Publication number: **0 496 233 A1**

# (12) EUROPEAN PATENT APPLICATION

(21) Application number: **92100405.7**

(51) Int. Cl.[5]: **A61K 31/725**, A61K 9/48

(22) Date of filing: **13.01.92**

Priority number: MI 91 A 000105.

(30) Priority: **18.01.91**

(43) Date of publication of application:
**29.07.92 Bulletin 92/31**

(84) Designated Contracting States:
**AT BE CH DE DK ES FR GB GR IT LI LU MC NL PT SE**

(71) Applicant: **SCLAVO S.p.A.**
**Via Fiorentina 1**
**I-53100 Siena(IT)**

(72) Inventor: **Lattanzi, Filippo**
**Via A. Sansedoni, 9**
**I-53100 Siena(IT)**

(74) Representative: **Gervasi, Gemma, Dr. et al**
**Studio Brevetti e Marchi NOTARBARTOLO & GERVASI 33, Viale Bianca Maria**
**I-20122 Milano(IT)**

(54) **Pharmaceutical compositions for oral use containing low molecular weight heparin, their preparation and use.**

(57) Disclosed are pharmaceutical compositions containing as the active principle low molecular weight heparin useful, for oral administration.

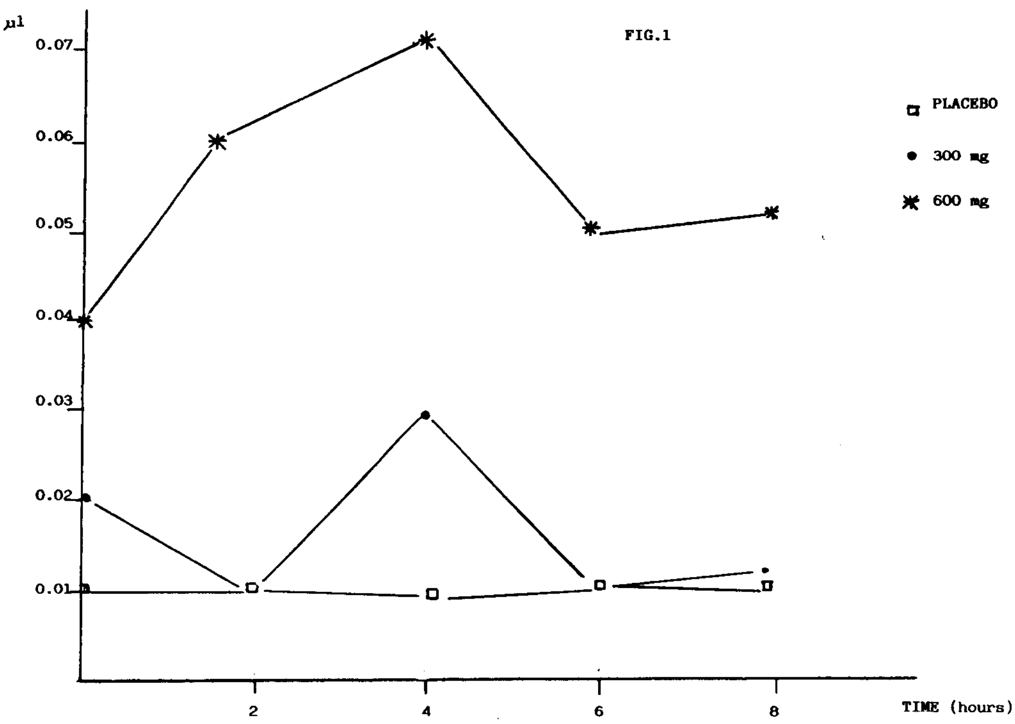


EP 0 496 233 A1

Field of the invention

Disclosed are pharmaceutical compositions for oral use containing as the active principle low molecular weight heparins, or their pharmaceutically acceptable salts.

Prior art

It is known that mucopolysaccharides, as well the proteic drugs, cannot be administered by oral route, since they are easily attacked by the gastric juices and by the enzymes present in the stomach.

For this reason, heparin and its derivatives can be administered only by parenteral (intramuscular, intravenous and subcutaneus) route.

It is also known since its anticoagulant activity was ascertained, that heparin, which is a mucopolysaccharide having a molecular weight ranging from 6 to 20,000 Daltons normally present in the mammal tissues and particularly in liver and in lungs, is useful for thrombosis treatment.

In fact it has been found that heparin anticoagulant activity is due to its inhibition action towards thrombin and other factors forming the complex coagulation mechanism, while its antithrombotic activity implies the inhibition of another factor, namely the Xa-Factor.

In the last years it has been repeatedly demonstrated that heparin anticoagulant activity decreases in proportion to the decrease of its molecular weight while its anti-Factor Xa activity is independent of its molecular weight. It is therefore evident that low molecular weight heparins can be considered as potential effective antithrombotic agents having low risks of hemorrhagic complications. In fact several clinical studies published by persons skilled in the field of thrombosis have demonstrated that low molecular weight heparin can be useful to prevent post-surgical deep venous thrombosis. All over the world various low molecular weight heparins were prepared in which the advantageous requisites (anti thrombotic activity and possible antilipemic activity) were exalted and the less desired properties (anticoagulant activity and therefore hemorrhagic and thrombocytopenic complications) were instead reduced.

In European Patent Application EP-A-116 801 here reported as a reference, a low molecular weight heparin is disclosed as well its pharmaceutically acceptable salts and a process for its preparation. This heparin having a molecular weight comprised between 2,000 and 9,000 and a sulfation degree at least of 2.5, called Supersulfated Heparin (SSH), can be represented by formula (I):

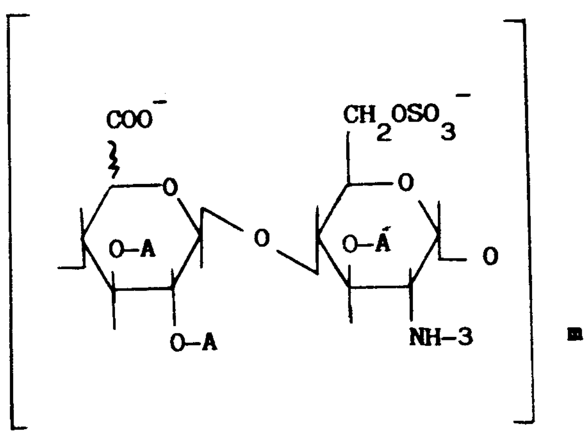

(I)

wherein A is H or $SO_3^-$, B is $SO_3^-$ or $COCH_3$, m is comprised between 4 and 15 and the bond indicates that the group $COO^-$ may have D or L configuration.

Among the pharmaceutically acceptable salts, we can consider for example those with alkali- and alkali-earth metals, such as sodium, potassium, calcium, magnesium and similar ones.

The above mentioned heparin, has been found to meet the desired requirements and has become in use as the active principle for the formulation of pharmaceutical compositions for parenteral use.

It is evident that it would be extremely interesting to dispose, besides of a parenteral formulation, also of an oral formulation that would be more suitable for a prolonged treatment, namely for a treatment not only intended to intervene only in the acute episodes, but also a treatment intended to control a pathologic chronic condition in order to avoid the occurrence of said acute episodes; said prolonged treatment can be for example the treatment of occluding artheriopathy of the lower extremities, the treatment of chronic

venous insufficiency, etc.

Detailed description of the invention

The present invention allows to answer effectively to the above mentioned requirement.

In fact it has been unexpectedly found that mixing a low molecular weight heparin, or its pharmaceutically acceptable salts, with suitable excipients, in hard gelatin capsules coated with a gastro-resistant layer, a marked anti-Xa activity is observed without any thrombin generation effect, which fact being not predictable since the previous knowledge on the subject, as above said, suggested only a parenteral use for heparins.

The pharmaceutical compositions according to the present invention are effective for preventing the deep venous thrombosis (anti-Xa activity) and are practically devoid of anticoagulant activity.

The activity of the pharmaceutical compositions according to the invention has been proved as far as the hemocoagulative effect is concerned by means of the following tests:

- APTT (Activated Partial Thromboplastine Time)
- Anti-Xa activity (Anti factor X activated)
- Anti-IIa activity (Anti factor II activated)

while as far as the activating effect on the fibrinolytic system is concerned, by means of the following tests:

- Determination of the histic activity plasmatic values of the plasminogen (t-PA = tissue Plasminogen Activator) and of the activator inhibitor (PAI = Plasminogen Activator Inhibitor) by means of the chromogenic method.
- Determination of the lysis time of diluted blood (WDBLT = whole diluted Blood Lysis Time) by means of the Chohen and others method.

It was thus proved that the good anti-Xa activity (antithrombotic activity) of the compositions according to the present inventions is followed by an almost complete lack of activity of the molecules on APTT with the contemporaneous advantage of a good effectiveness in the lysis time of the euglobulines (fibrinolytic activity).

In order to prepare the compositions according to the present invention, both the low molecular weight heparin and the excipients (maize starch, talc, magnesium stearate, lactose and similar ones) are ground and if necessary sieved and dried. The exact necessary quantity of every component for the preparation of the lot are weighed and mixed for 15 minutes in a suitable powder mixer. The hard gelatin capsules are then filled checking at scheduled times that the content weight be the desired one. The test for the weight uniformity is therefore carried out and capsules are sent to coating. This is accomplished by acid pH resistant materials based on copolymers of methacrylic acid and its esters, or other suitable materials.

After coating, the capsules are subjected to the disaggregation test according to the Official Pharmacopoeia (OP) IX Ed. and then to all the checks encompassed in the usual procedure.

The quantity of the active principle in the compositions may range from 50 to 500 mg of SSH, preferably from 150 to 400 mg.

The quantity of excipients is normally comprised between 50 and 300 mg, preferably between 60 and 200 mg.

EXAMPLE 1

Hard gelatin capsules of adequate capacity coated with a gastro-resistant layer in order to prevent the gastric juices attack, were filled with the following formulation expressed as an unitary dosage:

| | |
|---|---|
| SSH | 150 mg |
| Maize starch | 83 mg |
| Talc | 67 mg |
| Magnesium stearate | 3 mg |

Capsules were administered to 5 healthy voluntaries and the results are shown in Fig. 1 wherein on the abscissa axis the reaction times are reported and on the ordinate axis the anti Xa effect expressed in µl.

EXAMPLE 2

Hard gelatin capsules of suitable capacity coated with a gastro resistant layer, were filled with the

following formulation expressed as unitary dosage:

| | |
|---|---|
| SSH | 400 mg |
| Anhydrous lactose | 76 mg |
| Magnesium stearate | 4 mg |

The following examples report other formulations according to the invention containing SSH as the active principle utilizable for oral administration in the form of capsules of gelatin coated with a gastro resistant layer.

The doses hereinafter shown refer to a formulation for the preparation of 1000 capsules.

| EXAMPLE 3 | |
|---|---|
| SSH | 150 g |
| Maize starch | 110 g |
| Lactose | 60 g |
| Talc | 15 g |
| Magnesium Stearate | 3 g |

| EXAMPLE 4 | |
|---|---|
| SSH | 400 g |
| Maize starch | 30 g |
| Lactose | 35 g |
| Magnesium stearate | 4 g |

| EXAMPLE 5 | |
|---|---|
| SSH | 300 g |
| Lactose | 175 g |
| Magnesium stearate | 4 g |

| EXAMPLE 6 | |
|---|---|
| SSH | 300 g |
| Maize starch | 75 g |
| Lactose | 85 g |
| Talc | 15 g |
| Magnesium stearate | 4 g |

| EXAMPLE 7 | |
|---|---|
| SSH | 150 g |
| Maize starch | 110 g |
| Lactose | 60 g |
| Magnesium stearate | 3 g |

| EXAMPLE 8 | |
|---|---|
| SSH | 150 g |
| Maize starch | 83 g |
| Talc | 67 g |
| Magnesium stearate | 3 g |

| EXAMPLE 9 | |
|---|---|
| SSH | 400 g |
| Lactose | 75 g |
| Magnesium stearate | 4 g |

## Claims

1. Pharmaceutical compositions for oral use containing as the active principle low molecular heparins or their pharmaceutically acceptable salts.

2. The compositions as claimed in claim 1 wherein the low molecular weight heparin is represented by a product having formula (I)

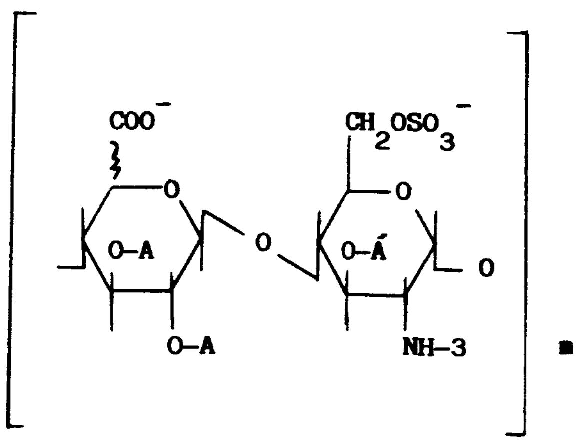

(I)

wherein A is H or $SO_3^-$, B is $SO_3^-$ or $COCH_3$, m is comprised between 4 and 15 and the ⌇ bond indicates that the group $COO^-$ may have D or L configuration; said heparin having a molecular weight comprised between 2,000 and 9,000 and a sulfation degree of at least of 2.5.

3. The compositions as claimed in claims 1 and 2 wherein the excipient is selected from: maize starch, talc, magnesium stearate, lactose, and their admixtures.

4. The compositions as claimed in claims 1-3 wherein the active principle is comprised between 50 and 500 mg.

5. The compositions as claimed in claim 4 wherein the active principle is comprised between 150 and 400 mg.

6. The compositions as claimed in claim 4 and 5 wherein the excipient is comprised between 50 and 300 mg.

**7.** The compositions as claimed in claim 6 wherein the excipient is comprised between 60 and 200 mg.

**8.** The composition as claimed in claims 1-7 in the form of gastroresistant hard gelatin capsules.

**9.** The use of compositions as claimed in claims 1-7 for thrombosis treatment.

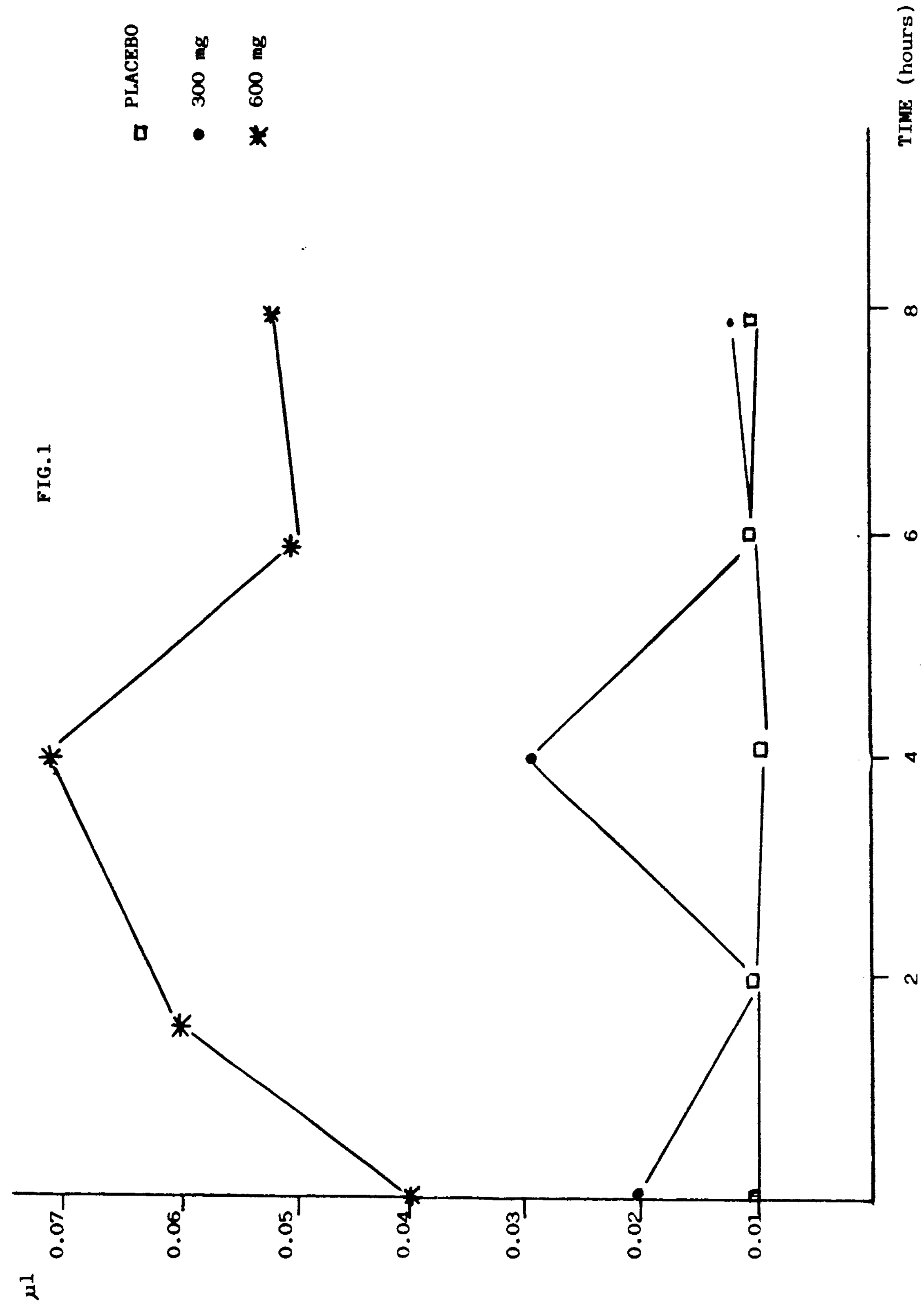
PLACEBO
300 mg
600 mg
µl
0.07
0.06
0.05
0.04
0.03
0.02
0.01
2
4
6
8
TIME (hours)

FIG.1

European Patent Office

# EUROPEAN SEARCH REPORT

Application Number

EP 92 10 0405

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 116 801 (ANIC)<br>* Claims; page 11, lines 8-30 *<br>--- | 1-9 | A 61 K 31/725<br>A 61 K 9/48 |
| P,X | EP-A-0 432 537 (IKETON)<br>* Claims; page 4, lines 23-39 *<br>----- | 1-9 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 13-03-1992 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)